# EUROPEAN PATENT SPECIFICATION

(11) **EP 2 033 593 B1**
(45) Date of publication and mention of the grant of the patent: **31.10.2012**
(21) Application number: 08163752.2
(22) Date of filing: 05.09.2008
(51) Int. Cl.: A61B 19/00, A61F 2/24

(54) **"Microprocessor controlled delivery system for cardiac valve prosthesis"**
Mikroprozessorgesteuertes Ausgabesystem für eine Herzklappenprothese
Système de livraison contrôlée à microprocesseur pour prothèse valvulaire cardiaque

(30) Priority: 15.05.2008 US 53570 P; 07.09.2007 EP 07115951; 07.09.2007 EP 07115960
(43) Date of publication of application: 11.03.2009
(73) Proprietor: SORIN BIOMEDICA CARDIO S.R.L., 13040 Saluggia (Vercelli) (IT); Mayo Foundation for Medical Education and Research, Rochester, MN 55905 (US)
(72) Inventor: Ziarno, Andrew W., 20159, Milano (IT); Suri, Rakesh M., Rochester, MN 55902 (US); Manasse, Eric, 20123, Milano (IT)
(74) Representative: Bosotti, Luciano

(56) References cited:
- EP-A- 1 935 377
- WO-A-01/21244
- WO-A-2005/046525
- WO-A-2005/065200
- WO-A-2006/135551
- WO-A-2007/076463
- US-A1- 2006 100 639

## Description

This application claims the benefit of U.S. provisional application No. 61/053,570, entilted "Microprocessor Controlled Delivery System for Cardiac Valve Prothesis," filed May 15, 2008, and European Applications Nos. 07115960.2 and 071151.1 filed September 7, 2007.

### TECHNICAL FIELD

The present invention relates to instruments for the in situ delivery and positioning of implantable devices. In particular, the invention relates to the in situ delivery of expandable prosthetic cardiac valves using a microprocessor controlled delivery system.

### BACKGROUND

Recently, there has been increasing consideration given to the possibility of using, as an alternative to traditional cardiac valve prostheses, valves designed to be implanted using minimally-invasive surgical techniques or endovascular delivery (so-called "percutaneous valves"). Implantation of a percutaneous valve (or implantation using thoracic-microsurgery techniques) is a far less invasive act than the surgical operation required for implanting traditional cardiac valve prostheses.

These expandable prosthetic valves typically include an anchoring structure or armature, which is able to support and fix the valve prosthesis in the implantation position, and prosthetic valve elements, generally in the form of leaflets or flaps, which are stably connected to the anchoring structure and are able to regulate blood flow. One exemplary expandable prosthetic valve is disclosed in U.S. Publication 2006/0178740 A1.

An advantage of these expandable prosthetic heart valves is that they enable implantation using various minimally invasive or sutureless techniques. One non-limiting exemplary application for such an expandable valve prosthesis is for aortic valve replacement. Various techniques are generally known for implanting such an aortic valve prosthesis and include percutaneous implantation (e.g., transvascular delivery through a catheter), dissection of the ascending aorta using minimally invasive thoracic access (e.g., mini-thoracotomy), and transapical delivery wherein the aortic valve annulus is accessed directly through an opening near the apex of the left ventricle. Note that the percutaneous and thoracic access approaches involve delivering the prosthesis in a direction opposing blood flow (i.e., retrograde), whereas the transapical approach involves delivering the prosthesis in the same direction as blood flow (i.e., antegrade) Similar techniques may also be applied to implant such a cardiac valve prosthesis at other locations (e.g., a pulmonary valve annulus).

More specifically, the invention reletes to a devices according to the preamble of claim 1, which is known e.g. from WO 2005/065200 A.

### SUMMARY

The present invention, having the features set forth in claim 1 according to one embodiment, is a device for implanting an expandable heart valve prosthesis, the device comprising a deploying mechanism capable of deploying the prosthesis and a microprocessor communicatively linked with at least a portion of the deploying mechanism.

It is also given an example of a method of deploying an expandable heart valve prosthesis, the method comprising deploying the prosthesis using a microprocessor controlled delivery device.

According to another embodiment, the present invention is a device for implanting a heart valve prosthesis, the device comprising a microprocessor and at least one functionality controlled by the microprocessor.

An example of an improved method of delivering an implantable heart valve prosthesis is also disclosed. The improvement comprising superimposing real-time images taken from an imaging mechanism onto pre-operatively taken three-dimensional images and positioning the prosthesis as a function of its location in relation to the images.

While multiple embodiments are disclosed, still other embodiments of the present invention will become apparent to those skilled in the art from the following detailed description, which shows and describes illustrative embodiments of the invention. As will be realized, the invention is capable of modifications in various obvious aspects, all without departing from the present invention as defined by the claims. Accordingly, the drawings and detailed description are to be regarded as illustrative in nature and not restrictive.

### BRIEF DESCRIPTION OF THE DRAWINGS

Figures 1A and 1B illustrate, in general terms, a delivery instrument according to two exemplary embodiments of the present invention.

Figure 2 is a partial cutaway, perspective view of a distal portion of the instrument of Figure 1, according to one embodiment of the present invention.

Figures 3A-3E illustrate a sequence of deploying a prosthetic heart valve using a retrograde approach, according to one embodiment of the present invention.

Figures 4A-4E illustrate a sequence of deploying a prosthetic heart valve using an antegrade approach, according to another embodiment of the present invention.

Figures 5A-5C illustrate a sequence of deploying a prosthetic heart valve, according to yet another embodiment of the present invention.

Figures 6-9 illustrates further possible features of the instrument illustrated herein, according to various embodiments of the present invention.

Figure 10A-10D illustrate a sequence of deploying a prosthetic heart valve, according to another embodiment of the present invention.

Figure 11 is a schematic diagram of a control system for controlling deployment of a prosthetic heart valve.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

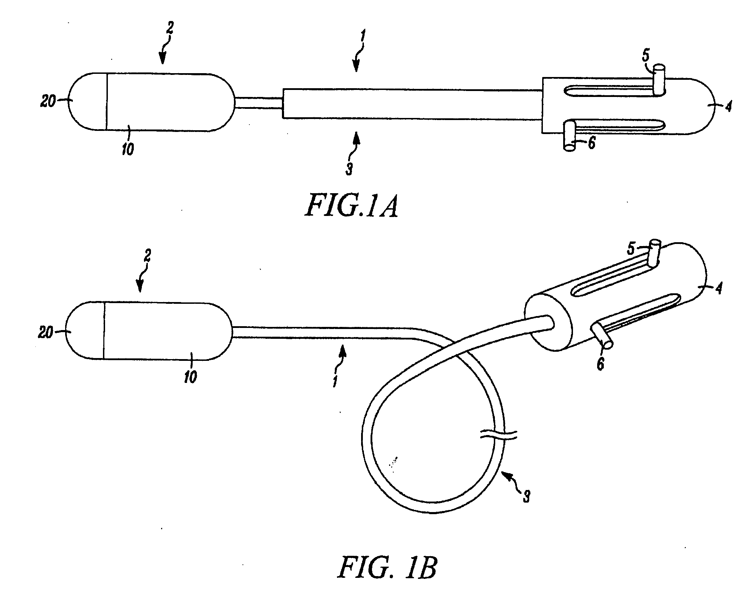

Figures 1a and 1b show an instrument 1 for implanting and radially deploying in situ an expandable, prosthetic cardiac valve. Purely by way of example, the prosthetic cardiac valve could be of the type described in U.S. Publication 2006/0178740 A1. As will be apparent to one skilled in the art, however, the instrument 1 could be used to deliver a variety of prosthetic cardiac valves and is not limited to any particular prosthetic valve structure.

As shown in Figure 1, the instrument 1 includes a carrier portion 2 for enclosing and carrying the prosthetic device and a manipulation portion 3 that couples the carrier portion 2 to a control handle 4 where two actuator members (for instance two sliders 5, 6) are located.

The manipulation portion 3 may assume various configurations. Figure 1A shows a configuration where the portion 3 is comprised of a substantially rigid bar with a length (e.g., 10 cm) that will permit positioning of the carrier portion 2, and the prosthetic cardiac valve carried thereby, at an aortic valve site. This configuration is adapted for use, for example, in the sutureless and the transapical implantation methods. Figure 1B, conversely, shows a second configuration, where the portion 3 is essentially comprised of an elongated, flexible catheter-like member that allows positioning of the carrier portion 3, and the prosthetic cardiac valve carried thereby, at an aortic valve site via transvascular catheterization (e.g., initiating at the femoral artery). This second configuration is also amenable for use in the sutureless or transapical implantation techniques. In one embodiment, the flexible, catheter-like member is braided or otherwise adapted to facilitate transmission of torque from the handle 4 to the carrier portion 2, such that the operator may effect radial positioning of the carrier portion 2 during the implantation procedure.

In one embodiment, the instrument 1 is adapted for use with a separate delivery tool. The instrument 1, for example, may be sized and shaped for delivery through a lumen of a tube or trocar during a "sutureless" or transapical delivery technique. Likewise, the instrument 1 may be adapted for delivery through a working lumen of a delivery or guide catheter. In this embodiment, for example, the operator may first deliver a guide catheter through the patient's vasculature to the implant site and then advance the instrument 1 through the lumen. In other embodiments, other techniques known in the art are used to reach the implantation site from a location outside the patient's body.

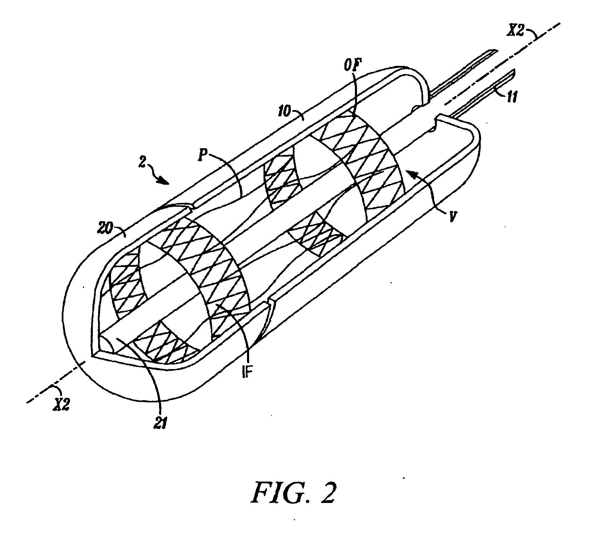

As shown in Figure 2, the carrier portion 2 includes two deployment elements 10, 20, each independently operable to allow the expansion of at least one corresponding, radially expandable portion of the implant device. In the case of the cardiac valve prosthesis, indicated as a whole as V, which is disclosed in U.S. Publication 2006/0178740 A1, two such radially expandable portions are provided situated respectively at the inflow end IF and the outflow end OF for the pulsated blood flow through the prosthesis. In alternative embodiments, however, the cardiac valve prosthesis may include more than two expandable members and, likewise, the carrier portion 2 may include more than two independent deployment elements. The valve prosthesis may be self-expanding (e.g., made from a superelastic material such as Nitinol) or may require expansion by another device (e.g., balloon expansion).

Figure 2 illustrates an embodiment for use with a self-expanding cardiac valve prosthesis. As shown in Figure 2, the cardiac valve prosthesis V is arranged within the carrier portion 2, such that an expandable portion IF and an expandable portion OF are each located within one of the deployment elements 10, 20. Each deployment element 10, 20 may be formed as a collar, cap or sheath. Each deployment element 10, 20 is able to constrain the portions IF, OF in a radially contracted position, against the elastic strength of its constituent material. The portions IF, OF are able to radially expand, as a result of their characteristics of superelasticity, only when released from the deployment element 10, 20. Typically, the release of the portions IF, OF is obtained by causing an axial movement of the deployment elements 10, 20 along the main axis X2 of the carrier portion 2. In one embodiment, the operator (e.g., physician) causes this axial movement by manipulating the sliders 5 and 6, which are coupled to the deployment elements 10, 20.

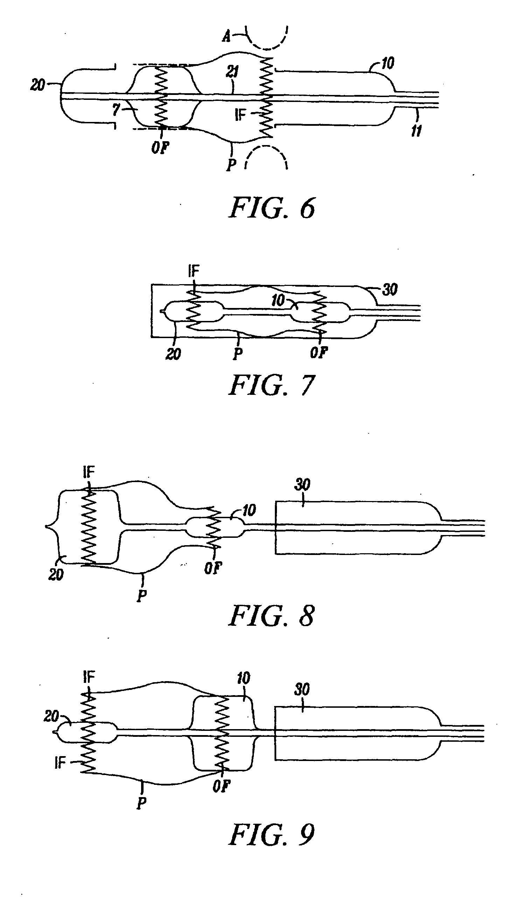

In an alternative embodiment (shown in Figures 7-9), expansion of the radially expandable portions IF, OF is caused by a positive expansion action exerted by the deployment elements 10, 20. In the embodiments shown in Figures 7-9, the deployment elements 10, 20 are comprised of expandable balloons onto which the portions IF, OF are coupled (e.g., "crimped") in a radially contracted position. In this embodiment, the operator causes radial expansion of the portions IF, OF by causing expansion of the balloons, using any of a variety of techniques known in the art.

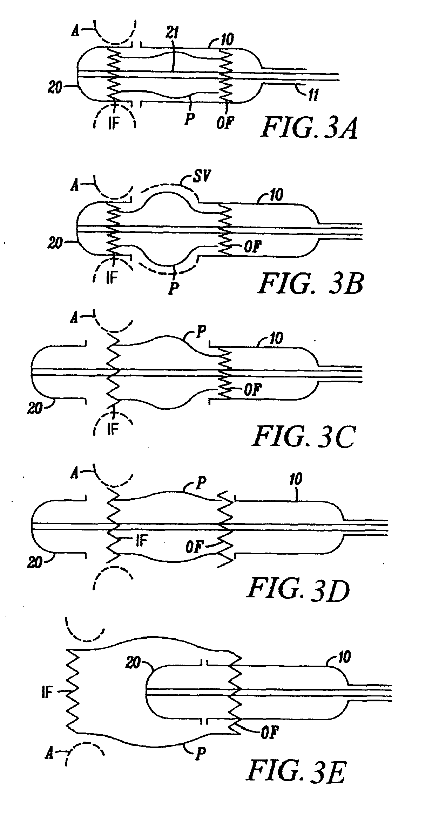

Figures 3-5 illustrate exemplary deployment techniques for the embodiment wherein the expandable portions IF, OF are made of a self-expandable material. In Figures 3-5, only the armature of the prosthetic cardiac valve prosthesis V is schematically shown (i.e., the valve leaflets are not shown). As shown, the armature includes the expandable entry (inflow) portion IF and the expandable exit (outflow) portion OF, which are connected axially by anchoring formations P. In one embodiment, as described in U.S. Publication 2006/0178740, the formations P are spaced at 120° intervals about the armature circumference and are configured to radially protrude from the prosthesis V so as to penetrate into the sinuses of Valsalva.

In the case of a cardiac valve prosthesis to be deployed at an aortic position, the inflow end IF of the prosthesis V is located in correspondence with the aortic annulus, thereby facing the left ventricle. The profile of the aortic annulus is shown schematically by the dashed lines A in Figures 3-5. Conversely, the outflow end OF is located in the ascending line of the aorta, in a position immediately distal to the sinuses of Valsalva, wherein the formations P extend.

Figures 3-5 show a carrier portion 2 having two deployment elements 10, 20 each of which is capable of "encapsulating" a respective one of the inflow IF and outflow OF portions, to constrain the portions IF, OF from radially expanding. Both of the elements 10, 20 can be arranged to slide longitudinally with respect to the principal axis X2 of the carrier portion 2. The axial movement of the elements 10, 20 is obtained, according to exemplary embodiments, via the sliders 5, 6 provided on the handle 4 at the proximal end of the manipulation portion 3 of the instrument 1. For instance, the slider 5 may act on the deployment element 20 through a respective control wire or tendon 21, while the slider 6 may act on the deployment element 10 through a tubular control sheath 11 slidably arranged over the tendon 21, with both the elements 11, 21 slidable along the axis X2.

In one exemplary embodiment, an internal surface of the elements 11, 21 comprise a low-friction or lubricious material, such as an ultra-high molecular weight material or PTFE (e.g., Teflon®). Such a coating will enable the elements 11, 21 to move or slide with respect to the portions IF, OF, such that the portions IF, OF are released upon axial movement of the elements 11, 21.

In one embodiment, the sheath 11 is movable in a distal-to-proximal direction, so that the sheath and thus the element 10 move or slide "backwards" with respect to the carrier portion 2. In a complementary manner, the sliding movement of the tendon 21 will take place in a proximal-to-distal direction, so that the tendon and thus the element 20 move or slide "forwards" with respect to the carrier portion 2. In another embodiment, movement of the elements 10, 20 is obtained by manipulating rigid actuation members from the handle 4.

Figures 3-5 are deliberately simplified for clarity of representation and do not take into in account, for instance, the fact that the portion 3 of the instrument may include other control tendons/sheaths and/or ducts for inflating the post-expansion balloons (see Figure 6). Also, the element 20 could be actuated by means of a sheath rather than a tendon. Also, whatever their specific form of embodiment, the actuator members 11, 21 of the deployment elements 10, 20 may also have associated locking means (not shown, but of a known type) to prevent undesired actuation of the deployment elements 10, 20.

Notably, the deployment elements 10, 20 are actuatable entirely independently of each other. This gives the operator complete freedom in selecting which of the portions IF, OF to deploy first according to the specific implantation method or conditions. Figures 3A-3E, for example, illustrate use of the instrument 1 for a "retrograde" approach (e.g., in the case of sutureless or percutaneous implantation), to the valve annulus, wherein the cardiac valve prosthesis V approaches the valve annulus from the aortic arch.

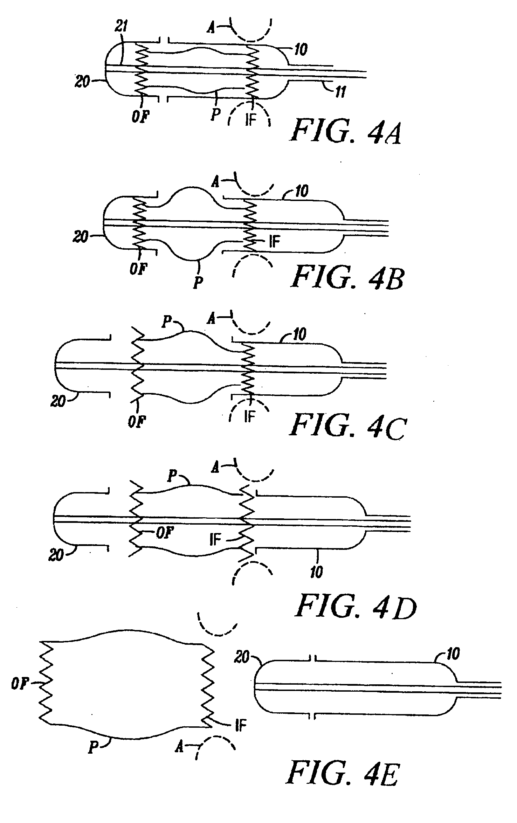

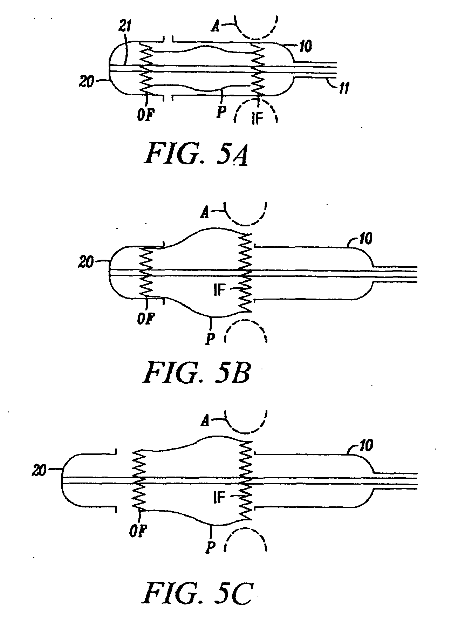

In Figure 3A (as in the following Figures 4A and 5A), the cardiac valve prosthesis V is shown mounted in or carried by the carrier portion 2 of the instrument 1, such that the deployment elements 10, 20 constrain the annular ends IF, OF of the prosthesis V in a radially contracted position.

Figure 3B shows the element 10 retracted axially with respect to the axis X2 of the carrier portion 2 a sufficient distance to uncover and release the formations P, which are then able to expand (e.g., due to their superelastic construction) such that they protrude beyond the diameter of the elements 10, 20. As shown in Figure 3B, the formations P are allowed to expand, while the remaining portions of the prosthesis V are maintained in a radially contracted configuration. In the configuration shown in Figure 3B, the operator can take the necessary action for ensuring the appropriate positioning of the prosthesis V in correspondence with the sinuses of Valsalva SV. The profile of the sinuses of Valsalva are shown schematically in Figure 3B by the dashed lines SV.

Such appropriate positioning includes both axial positioning (i.e. avoiding deploying the prosthetic valve V too far "upstream" or too far "downstream" of the desired position with the ensuing negative effect that the inflow end IF is not correctly positioned with respect to the valve annulus A) and radial positioning. The sinuses of Valsalva are configured as a hollow, three-lobed structure. Accordingly, accurately positioning each formation P of the prosthesis V in a respective sinus of Valsalva will ensure the correct positioning or angular orientation of the prosthetic valve as a whole, which will ensure that the leaflets of the prosthetic valve are correctly oriented (i.e., extend at the angular positions of the annulus where the natural valve leaflets were located before removal).

In exemplary embodiments, the instrument 1 may further include various structures or features to assist the operator in obtaining the appropriate axial positioning with respect to the aortic annulus and radial positioning with respect to the sinuses of Valsalva. The instrument 1 (or the guide catheter or delivery tube), for example may include a lumen sufficient to allow the injection of contrast fluid to a location at the implantation site. For the embodiment shown in Figure 3, for example, this lumen would have an opening located past the inflow end IF or the prosthesis V, such that any injected contrast fluid would then flow back toward the prosthesis V, thereby enabling the operator to obtain a visual image of the implantation site, including an image of the sinuses of Valsalva. Likewise, in other embodiments, the prosthesis V may include radiopaque markers disposed at appropriate locations to assist in this positioning.

In one exemplary embodiment (e.g., in the case of "sutureless" implantation), the carrier portion 2 and the prosthesis V may be arranged from the beginning in the configuration represented in Figure 3B, namely with the formations P already protruding radially with respect to the profile of the prosthesis, while the annular end portions IF, OF are constrained in a radially contracted position by the elements 10, 20. In this case, the element 10 will have a sufficient length only to cover the axial extension of the annular end portion OF, as it need not radially constraint the formations P.

Figure 3C shows the element 20 displaced distally with respect to the prosthesis V by the tendon 21. As shown, the element 20 was displaced a length sufficient to uncover the annular inflow portion IF, such that the portion IF is able to expand radially to assume the desired anchoring position at the valve annulus A. This release of the inflow portion IF takes place while the prosthetic valve V is still precisely retained and controlled by the instrument 1, such that it will not move or "jump" with respect to the valve annulus during the expansion of the portion IF.

It will also be appreciated that from the configuration shown in Figure 3C, the operator may return to the configuration shown in Figure 3A, so as to cause a radial contraction of the formations P and, even if in an incomplete manner, of the annular inflow portion IF. This will allow the operator to withdraw the prosthesis V from the implantation site if the operator believes that the implantation procedure has thus far not yielded a satisfactory result.

Next, the prosthetic implantation process progresses by sliding the deployment element 10 so that it releases the outflow annular portion OF. The portion OF can then radially expand against the aortic wall, thus completing the second phase of the implantation operation of the prosthesis V.

Finally, as shown in Figure 3E, the carrier portion 2 and the instrument 1 as a whole can be withdrawn with respect to the implantation site through the center of the prosthesis V. In one embodiment, the carrier portion 2 is withdrawn after the deployment elements 10, 20 have been brought back to their initial positions, that is after having caused the elements 10, 20 to slide, in a proximal-to-distal and in a distal-to-proximal direction, respectively. The sequence of operations represented in Figures 3A-3E may be accomplished with a pulsating heart and without interrupting the natural circulation of blood.

Figures 4A-4E show an implantation procedure of a prosthesis V, according to another embodiment of the present invention. This procedure is similar to the procedure shown in Figures 3A-3E, but Figures 4A-4E show an "antegrade" approach, typical of a transapical implantation procedure. In this case, the prosthesis V (mounted in the carrier portion 2) is advanced to the implantation site (e.g., aortic valve) through the left ventricle. While reference is again made herein to a prosthetic valve for the substitution of the aortic valve, the same criteria and principles will also apply to different valve types (e.g. mitral). Various techniques for accessing the aortic valve site through the left ventricle are known in the art. One exemplary technique for transapical delivery is disclosed in U.S. Publication 2005/0240200.

Figures 4A-4E are substantially identical to Figures 3A-3E, except that the position assumed by the prosthetic valve V is inverted. Accordingly, in the case of the intervention of "antegrade" type of Figures 4A-4E, the carrier portion 2 of the instrument 1 with the prosthesis V mounted therein is passed completely through the valve annulus A, so as to position the inflow portion IF in correspondence with the valve annulus A.

After withdrawing the deployment element 10, so as to release the formations P (Figure 4B), the deployment element 20 is advanced distally, so as to release and allow the outflow annular end portion OF to radially expand against the aortic wall downstream of the sinuses of Valsalva (see Figure 4C). At this point, the operator is still in a position to ensure that the prosthesis has the required correct angular position by making sure that the formations P each correctly engage a corresponding sinus. If the formations P do not properly align with the sinuses of Valsalva, the operator may use the instrument to apply a torque to the prosthesis V, thereby causing a rotation of the prosthesis V into the proper angular position. In one exemplary embodiment, the tendon 21 includes a stop (not shown) configured to prohibit axial motion of the inflow portion IF. This stop may help prevent axial movement of the inflow portion IF during distal motion of the of the deployment element 20, thereby ensuring that the outflow portion OF is released before the inflow portion IF.

Subsequently, by completely withdrawing in a proximal direction the deployment element 10, the operator releases the annular inflow portion IF that is thus deployed in correspondence with the aortic valve annulus thus completing the two-step implantation procedure of the prosthetic valve V (see Figure 4D). Then, according to one embodiment, the procedure progresses by bringing the deployment elements 10, 20 back towards their initial position with the ensuing retraction of the instrument 1 from the inflow portion IF of the valve (Figure 4E).

Figures 5A-5C, which correspond closely to the sequence of Figures 4A-4C, show that (also for a procedure of the "antegrade" type) it is possible to effect the two-step implantation sequence of Figures 4A-4E by deploying the end portions IF and OF of the prosthetic valve V in the reverse order. In the technique of Figures 5A-5C, once the desired "axial" position is reached (as represented in Figure 5A, which is practically identical to Figure 4A) with the expandable inflow end IF in correspondence of the aortic valve annulus A, the inflow portion IF is expanded first by operating the deployment element 10 to release the corresponding inflow portion IF.

The implantation procedure then proceeds, as schematically represented in Figure 5C, with the second step of this two-step procedure, namely with the deployment element 20 advanced distally with respect to the prosthesis V so as to release the expandable outflow portion OF. The outflow portion OF is thus free to expand against the aortic wall in a region downstream of the sinuses of Valsalva into which the formations P protrude.

The teaching provided in Figures 5A-5C also apply in the case of a "retrograde" procedure, as shown in Figures 3A-3E. Because the deployment elements 10, 20 are adapted to be activated entirely independently of each other, the operator is free to choose the most suitable deployment sequence (inflow first and then outflow; outflow first and then inflow) as a function of the specific conditions of intervention. This sequence may be entirely independent of access to the implantation site being of the retrograde or antegrade type.

Figures 6 and 7 schematically illustrate embodiments in which the carrier portion 2 of the instrument 1 includes a balloon 7 at locations corresponding to at least one or to both annular ends of the cardiac valve prosthesis V. This balloon may be of any known type (e.g. of the type currently used in expanding stents or the like in a body lumen, which therefore does require a detailed description to be provided herein) and is intended for use in realizing a "post-expansion" of the corresponding end portion IF, OF of the prosthesis V, so as to radially urge it against the wall of the implantation lumen. For instance, as shown in Figure 6, the balloon 7 can be selectively expanded (by inflating it with well known means and criteria) in such a way as to produce a radial expansion of the expandable portion associated therewith (here the end portion OF).

This technique may be useful to avoid movement or "jumping" of the prosthesis V during implantation. For instance, if the operator fears that deployment of the inflow end portion IF in correspondence of the aortic annulus A may give rise to an undesired longitudinal displacement of the valve prosthesis V as a whole, while the inflow portion IF is being released by the element 10 and expands to engage the aortic annulus A, a post-expansion balloon 7 associated with the outflow end OF can be inflated. In this way, as long as the post-expansion balloon 7 is kept dilated, the outflow end OF is urged and thus safely anchored to the lumen wall and any undesired displacement of the prosthetic valve V in an axial direction is prevented. Once the inflow portion IF is safely positioned at the aortic annulus A, the balloon 7 can be deflated and the instrument 1 withdrawn.

Figures 7, 8 and 9 schematically illustrate, without the intent of making any specific distinctions between "antegrade" and "retrograde" approaches and any specific choice as to which end portion, inflow IF or outflow OF, is to be deployed first, that the same two-step mechanism for independently deploying the two end portions IF, OF illustrated in Figures 3, 4 and 5 can be implemented in the case of prostheses V including end portions IF, OF whose radial expansion is obtained via a positive outward expansion action exerted by means of deployment elements 10, 20 altogether constituted by expandable balloons. These may be balloons of any known type and substantially correspond, from a structural point of view, to the post-expansion balloons (see for instance see the balloon 7 of Figure 6) to which reference has been made previously.

Other embodiments of the present invention include "hybrid" solutions, where a cardiac valve prosthesis V includes one or more self-expandable portions (having associated deployment elements 10, 20 of the type illustrated in Figures 2-5) as well as and one or more portions radially expandable via an expandable deployment element (such as a balloon as illustrated in Figures 7-9).

In the case where expansion due to a positive action of one or more balloons is preferred over the use of a self-expandable portions, the same balloon may be used both as an expansion balloon (Figures 7, 8 and 9), and as a post-expansion balloon (Figure 6).

As schematically illustrated in Figures 7-9 (the same solution can be adopted also in the case of Figures 2-6, it is possible to provide a tubular sheath 30 that surrounds in the manner of a protective tunic the assembly comprised of the carrier portion 2 with the prosthetic valve V mounted therein. This with the purpose of facilitating, typically in a percutaneous implantation procedure, the advancement towards the implantation site through the tortuous paths of the vasculature of the patient without risks of undesired jamming or kinking. It will be appreciated that, for the same goal, the deployment elements 10, 20 normally exhibit a "streamlined" shape, exempt from protruding parts and/or sharp edges. This is particularly the case for the element 20 located at a distal position, which typically exhibits an ogive-like shape.

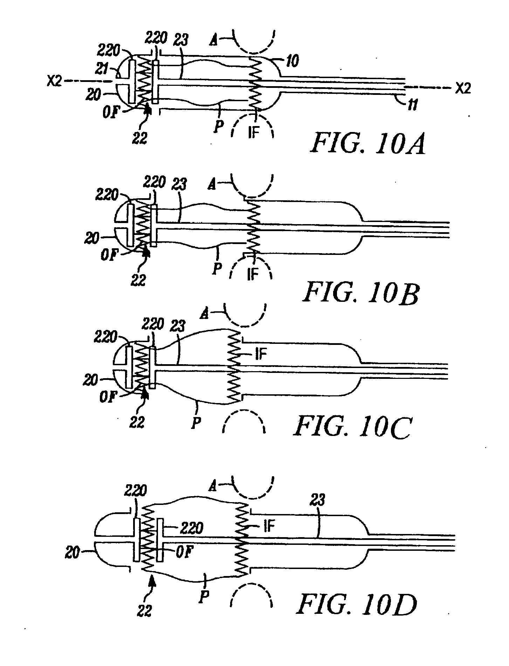

Figures 10A-10D, which substantially corresponds to Figures 5A-5C, illustrate an embodiment associating with either or both of the annular end portions IF, OF of the prosthesis V an "anti-skid" locking member 22. This member is primarily intended to prevent any undesired sliding movement of the end portion (IF and/or OF) with respect to its deployment element lengthwise of the carrier portion 2. Such a locking member is preferably associated with (at least) the annular end portion to be deployed second in the two-step deployment process of the prosthetic valve V described herein.

In this exemplary embodiment, the locking member 22 takes the form of a hub positioned at the distal end of a tubular member 23 having the wire 21 slidably arranged therein. The sheath 11 surrounds the tubular member 23 and is adapted to slide thereon so that the locking member 22 is capable of maintaining at a fixed axial position (e.g. via end flanges 220) the annular outflow portion OF with which the locking member is associated. The annular end portion in question is thus prevented from sliding axially of the deployment element 20, at least as long as the annular end portion OF is radially constrained by the deployment element 20.

The arrangement described makes it possible to adjust the position of the annular end portion locked by the locking member (and the position of the valve prosthesis V as a whole) both axially and angularly to the implantation site. This applies more or less until the annular portion expands to the point where further displacement is prevented by engagement of the annular portion with the valve annulus or the aortic wall. Additionally, the presence of the locking member(s) 22 facilitates possible recovery of the prosthetic valve V in case the implantation procedure is to be aborted.

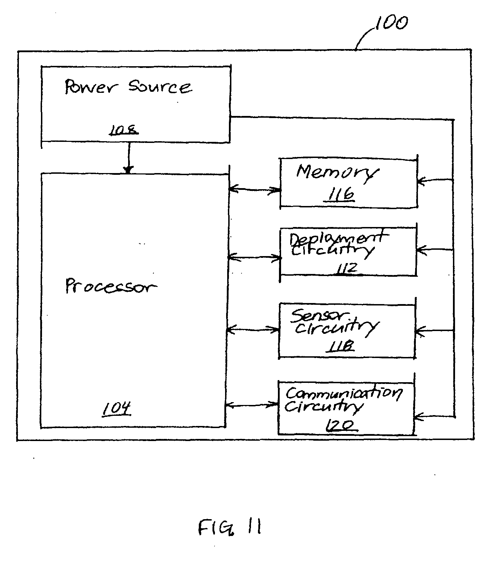

In one embodiment, the movement of the elements 10, 20 is controlled by a control system 100. As shown in Figure 11, according to an exemplary embodiment, the control system 100 includes a microprocessor or controller 104, a power source (e.g., battery) 108, deployment circuitry 112, memory 116, sensor circuitry 118, and communication circuitry 120. The control system 100 is enclosed in a hermetically sealed housing or capsule. In some embodiments, the control system 100 is embedded in the body of the delivery system catheter. It may, for example, be located in a lumen of the catheter. The microprocessor 104 may be of any type known in the art and suitable for incorporation into the instrument 1. The microprocessor 104 may, for example, be a multi-core processor as is known in the art.

In one embodiment, the control system 100 operates to actuate or control the deployment mechanism. In this embodiment, the control system 100 may receive instructions or commands from an operator or from an external system or device using the communication circuitry 120. Any of a variety of communication techniques known in the art may be employed, including for example, wireless communication (e.g., radio-frequency, inductive, and the like). Exemplary external systems may include an external image display system or anesthesia monitoring equipment.

The deployment circuitry 112 may include instructions (e.g., software) for optimal deployment of the cardiac valve prosthesis from the carrier portion 2. It may for example provide instructions to microactuators (e.g., an electric motor) coupled to the deployment elements 10, 20. The instructions may be configured to deploy the cardiac valve prosthesis using any of the techniques described above. According to one exemplary embodiment, the instrument 1 further includes a sensor coupled the sensor circuitry 118. The sensor is of any type generally known in the art for detecting a physiological parameter in the vasculature. A wide variety of sensor may be incorporated into the instrument 1, including for example a calcium sensor, a fluorescence sensor, a blood gas sensor, an oximetry sensor, and a cardiac output sensor. The sensor may, for example, be a pressure sensor configured for detecting the hemodynamics (e.g., pressure, flow rate, and the like) in the vasculature (e.g., the aorta) or in a heart chamber. According to various embodiments, the sensor provides a signal to the control system 100, which in turn processes this signal and uses the information to optimize deployment of the cardiac valve prosthesis.

According to other embodiments, the control system 100 further includes imaging circuitry. In this embodiment, the instrument 1 includes an imaging device or module configured to provide a signal indicative of a position within the vasculature or heart. The imaging device could, for example, be configured to detect proximity to a valve annulus (e.g., the aortic valve annulus). The imaging device, according to other exemplary embodiments, may also be used to generate any of the following visual images: the location of the prosthesis in a beating heart, a portion of the device in relation to anatomical structures in a patient's heart, the prosthesis in a stage of partial deployment, and the prosthesis in a fully deployed state. The imaging device may also generate an image of the annulus, which enables the control system 100 to determine the efficiency or effectiveness of annular debridement or native valve leaflet removal.

Any of a variety of suitable imaging devices may be included in the instrument 1, including for example an echocardiographic imaging module or an optical coherence tomography module. As is generally known, these systems are capable of providing an image in a vessel or heart chamber containing blood. The imaging system, according to other embodiments includes a magnetic resonance imaging (MRI) system, a stereotactic system, or a radiation-emitting chip. According to some embodiments, the imaging module is used to generate a digital image, which is then communicated to (and optionally stored in) the control system 100. This digital image may be used by the microprocessor to optimize deployment of the valve prosthesis. The imaging generated by the imaging module may, for example, be used by the control system 100 to optimize deployment of the valve prosthesis with respect to the native valve annulus. In one embodiment, the communication circuitry 120 is employed to transmit the digital image to an external device (e.g., a digital display). An operator (e.g., physician) may then use this display during manual implantation and deployment of the prosthetic cardiac valve. In the embodiment including the MRI imaging system, the MRI system may be configured to generate a real time image of the location of an MRI- compatible valve prosthesis with respect to the valve annulus. According to one embodiment, for example, real-time images generated by the imaging module are superimposed onto three-dimensional images (e.g., images generated pre-operatively). The user may then utilize these superimposed images to assist in guiding and positioning the prosthesis.

In another embodiment, the imaging system takes a three-dimensional image of an interior portion of a patient's arterial tree (e.g., the aortic arch) and at least a portion of a patient's heart. This imaging data is communicated to the control system 100. The microprocessor then processes the imaging data and determines the position of the valve prosthesis with respect to certain anatomical landmarks. The microprocessor then generates a drive signal to control the deployment or axial positioning of the valve prosthesis, to optimize placement. In one exemplary embodiment, the microprocessor actuates the deployment mechanism (using one of the techniques described in detail above), once the proximal end of the valve is located proximal to the aortic valve annulus. This sequence may be performed in an iterative function such that the imaging system continuously feeds real time image data to the control system and the microprocessor continuously optimizes deployment, such that ultimately the valve prosthesis is deployed at a location selected for optimal performance.

According to the invention, the instrument 1 includes a miniature pump of a type generally known in the art for pumping blood in a vessel or heart chamber. The pump may for example be any left ventricular assist device generally known in the art. In this embodiment, the pump is in communication with the control system 100. In one embodiment, the microprocessor receives a signal from a pressure or flow sensor and generates a pump drive signal based on this pressure or flow signal. In one exemplary embodiment, as flood flow decreases below a predetermined threshold, the microprocessor activates the blood pump. The instrument 1 includes, in another embodiment an injector configured to inject or release a therapeutic drug or medicament into the blood stream. The medicament may include for example a blood thinner (e.g., heparin). The injector may be communicably linked to the microprocessor, such that the microprocessor can activate the injector as appropriate (e.g., based on one or more signals received from the one or more sensors included in the instrument 1).

According to various embodiments, the instrument 1 further includes a module for native valve leaflet removal (i.e., leaflet debridement) or expansion (e.g., ballooning). Any of a variety of systems generally known in the art may be included with the instrument 1. In a further embodiment, the instrument 1 also includes a native valve annulus measuring device. In one implantation technique, the microprocessor controls the leaflet removal system, based on imaging data from the imaging device. Once valve removal (or ballooning) is complete, the measuring device measures the diameter of the valve annulus and communicates a signal indicative of this diameter to the control system. The microprocessor then uses this data to control deployment of the valve prosthesis. For example, the microprocessor controls the expansion balloon, such that it expands the valve prosthesis to an appropriate diameter for efficacious anchoring at the site of the valve annulus.

According to some embodiments, the instrument 1 includes a module adapted for leaflet removal or ballooning and also adapted for valve delivery. One such embodiment, for example, includes an inflatable expansion balloon (of the type well known in the art) operatively coupled to the manipulation portion 3 at a location either proximal or distal to the carrier portion 2. During operation, the user then positions the expansion balloon at or near the aortic valve annulus, such that the expansion balloon is generally adjacent to the native valve leaflets. The user then expands (e.g., by injecting an appropriate fluid) the expansion balloon sufficiently to expand the native valve leaflets and compress the leaflets against the annulus or the aorta. Alternatively, in embodiments including a valve removal system, the user operates the valve removal system to accomplish partial or complete removal of the native valve leaflets. Next, the user advances or retracts (as appropriate) the manipulation portion to place the carrier portion 2 at the appropriate location at or near the aortic valve annulus and operates the carrier portion 2 to deliver the prosthetic valve to the desired location. In some embodiments, the expansion balloon is sufficiently durable to enable expansion and compression of stenotic native valve leaflets.

According to various embodiments, the control system 100 of the present invention, is used to control operation of a valve delivery system such as that disclosed in U.S. Patent publication 2009/069 887 A1.. In other embodiments, the control system 100 is used to control operation of a valve positioning system, such as that disclosed in U.S. Patent publication 2008/147 160 A1. An in other embodiments, the control system 100 is used to control operation of a valve delivery system such as that disclosed in U.S. Patent publication 2009/069 886 A1.

According to various embodiments, the system of the present invention is used in conjunction with the various commercially available systems enabling robotic positioning, manipulation, and control of intravascular catheters. One such system, for example, is the Sensei™ Robotic Catheter System available form Hansen Medical based in Mountain View, California, USA. Other such exemplary systems are described in U.S. Patent Publication 2006/0276775 A1 and U.S. Patent Publication 2007/0250097 A1.

Various modifications and additions can be made to the exemplary embodiments discussed without departing from the scope of the present invention. Accordingly, the scope of the present invention as defined by the claims is intended to embrace all such alternatives, modifications, and variations as fall within the scope of the claims, together with all equivalents thereof as stipulated by Art. 69 EPC and the releted Protocol.

## Claims

1. A device for implanting an expandable heart valve prosthesis, the device (1) comprising a deployment mechanism (10, 20) capable of deploying the prosthesis and a microprocessor (100, 104) communicatively linked with at least a portion of the deployment mechanism (10, 20), **characterized in that** the device further comprises a pump communicatively linked to the microprocessor and a sensor and in which the pump variably pumps volumes of blood as a function of data from the sensor.

2. The device of claim 1 wherein the microprocessor (104) is configured to control operation of the deployment mechanism.

3. The device of claim 1 wherein the microprocessor (104) is a multi-core microprocessor.

4. The device of claim 1 wherein the device (1) further comprises a sensor (118) communicatively linked to the microprocessor (104).

5. The device of claim 4 wherein the sensor (118) is selected from the group consisting of a calcium sensor, a fluorescence sensor, a blood gas sensor, an oximetry sensor, and a cardiac output sensor.

6. The device of claim 1 further comprising an imaging module.

7. The device of claim 6 wherein the imaging module is a radiation emitting chip.

8. The device of claim 6 wherein the imaging module is selected from the group consisting of an echocardiographic imaging module and optical coherence tomography module capable of providing an image through blood.

9. The device of claim 6 further comprising communication circuitry (120) configured to transmit an image captured by the imaging module to an external device.

10. The device of claim 1 further comprising an injector, and wherein the injector is configured for control by the microprocessor.

11. The device of claim 2 wherein the deployment mechanism (10, 20) further comprises a microactuator.

12. The device of claim 2 wherein the deployment mechanism (10, 20) is capable of variably opening the expandable prosthesis.

13. The device of claim 2 further comprising a native valve removal or expansion mechanism operatively coupled to the deployment mechanism.

## Patentansprüche

1. Vorrichtung zur implantation einer expandierbaren Herzklappenprothese, umfassend einen Einsatzmechanismus (10, 20), der ausgebildet ist, um die Prothese einzusetzen, und einen Mikroprozessor (100, 104) der wenigstens mit einem Teil des Einsatzmechanismus (10, 20) kommunikativ verbunden ist, **dadurch gekennzeichnet, dass** die Vorrichtung weiterhin eine kommunikativ mit dem Mikroprozessor verbundene Pumpe und einen Sensor umfasst, wobei die Pumpe variierbar Blutvolumina als Funktion der Daten des Sensors pumpt.

2. Vorrichtung nach Anspruch 1, **dadurch gekennzeichnet, dass** der Mikroprozessor (104) ausgebildet ist, um den Betrieb des Einsatzmechanismus zu steuern.

3. Vorrichtung nach Anspruch 1, **dadurch gekennzeichnet, dass** der Mikroprozessor (104) ein Multi-Kern Mikroprozessor ist.

4. Vorrichtung nach Anspruch 1, **dadurch gekennzeichnet, dass** die Vorrichtung (1) weiterhin einen Sensor (118) umfasst, der kommunikativ mit dem Mikroprozessor (104) verbunden ist.

5. Vorrichtung nach Anspruch 4, **dadurch gekennzeichnet, dass** der Sensor (118) ausgewählt ist aus der Gruppe umfassend einen Kalzium-Sensor, einen Fluoreszenz-Sensor, einen Blut-Gas-Sensor, einen oximetrischen Sensor und einen Herzleistungs-Sensor.

6. Vorrichtung nach Anspruch 1, weiterhin umfassend ein Bildmodul.

7. Vorrichtung nach Anspruch 6, **dadurch gekennzeichnet, dass** das Bildmodul ein strahlungsemittierender Chip ist.

8. Vorrichtung nach Anspruch 6, **dadurch gekennzeichnet, dass** das Bildmodul ausgewählt ist aus der Gruppe umfassend ein echokardiografisches Bildmodul und ein optisches Kohärenz-Tomographie-Modul, welches ausgebildet ist, um durch Blut ein Bild bereitzustellen.

9. Vorrichtung nach Anspruch 6, weiterhin umfassend Kommunikations-Schaltkreise (120) die ausgebildet sind, um ein von dem Bildmodul erfasstes Bild an eine externe Vorrichtung zu übergeben.

10. Vorrichtung nach Anspruch 1, weiterhin umfassend einen Injektor, der ausgebildet ist, um von dem Mikroprozessor gesteuert zu werden.

11. Vorrichtung nach Anspruch 2, **dadurch gekennzeichnet, dass** der Einsetzmechanismus (10, 20) weiterhin einen Mikroaktuator umfasst.

12. Vorrichtung nach Anspruch 2, **dadurch gekennzeichnet, dass** der Einsetzmechanismus (10, 20) ausgebildet ist, um die expandierbare Prothese variabel zu öffnen.

13. Vorrichtung nach Anspruch 2, weiterhin umfassend einen Mechanismus zur Entfernung natürlicher Klappen oder einen Expansionsmechanismus, der operativ mit dem Einsetzmechanismus verbunden ist.

## Revendications

1. Dispositif pour implanter une prothèse valvulaire cardiaque expansible, le dispositif (1) comprenant un mécanisme de déploiement (10, 20) pouvant déployer la prothèse et un microprocesseur (100, 104) relié en communication avec au moins une partie du mécanisme de déploiement (10, 20), **caractérisé en ce que** le dispositif comprend en outre une pompe reliée en communication au microprocesseur et à un capteur, et dans lequel la pompe pompe de manière variable des volumes de sang en fonction des données provenant du capteur.

2. Dispositif selon la revendication 1, dans lequel le microprocesseur (104) est configuré pour contrôler le fonctionnement du mécanisme de déploiement.

3. Dispositif selon la revendication 1, dans lequel le microprocesseur (104) est un microprocesseur multicoeur.

4. Dispositif selon la revendication 1, dans lequel le dispositif (1) comprend en outre un capteur (118) relié en communication au microprocesseur (104).

5. Dispositif selon la revendication 4, dans lequel le capteur (118) est choisi dans le groupe consistant en un capteur de calcium, un capteur de fluorescence, un capteur de gaz du sang, un capteur d'oxymétrie, et un capteur de débit cardiaque.

6. Dispositif selon la revendication 1, comprenant en outre un module d'imagerie.

7. Dispositif selon la revendication 6, dans lequel le module d'imagerie est une puce d'émission de rayonnement.

8. Dispositif selon la revendication 6, dans lequel le module d'imagerie est choisi dans le groupe consistant en un module d'imagerie échocardiographique et un module de tomographie par cohérence optique capable de fournir une image à travers le sang.

9. Dispositif selon la revendication 6, comprenant en outre une circuiterie de communication (120) configurée pour transmettre à un dispositif externe une image prise par le module d'imagerie.

10. Dispositif selon la revendication 1, comprenant en outre un injecteur, et dans lequel l'injecteur est configuré pour être commandé par le microprocesseur.

11. Dispositif selon la revendication 2, dans lequel le mécanisme de déploiement (10, 20) comprend en outre un microactionneur.

12. Dispositif selon la revendication 2, dans lequel le mécanisme de déploiement (10, 20) est capable d'ouvrir de manière variable la prothèse expansible.

13. Dispositif selon la revendication 2, comprenant en outre un mécanisme de retrait ou d'expansion de valvule native, couplé de manière opérationnelle au mécanisme de déploiement.
